# EUROPEAN PATENT APPLICATION

(11) **EP 4 660 320 A1**
(43) Date of publication of application: **10.12.2025**
(21) Application number: 24750296.6
(22) Date of filing: 30.01.2024
(51) Int. Cl.: C12Q 1/6876, C12N 15/11, C12Q 1/686, C12Q 1/6813

(54) **METHOD AND KIT FOR DETECTING TARGET RNA**

(30) Priority: 31.01.2023 JP 2023013023
(71) Applicant: Toppan Holdings Inc., Tokyo 110-0016 (JP)
(72) Inventor: HIRASE, Takumi, Tokyo 110-0016 (JP); TAKAYA, Tomoyuki, Tokyo 110-0016 (JP); MAKINO, Yoichi, Tokyo 110-0016 (JP)
(74) Representative: Plasseraud IP
(86) International application number: PCT/JP2024/002871
(87) International publication number: WO 2024/162347

(57) **Abstract**

This method for detecting target RNA comprises: a step for introducing a reaction solution into a container, the reaction solution containing the target RNA, a reverse transcription reagent that reverse-transcribes the target RNA to generate target DNA, and a detection reagent that detects the target DNA; a step for bringing the reaction solution into a reverse-transcription condition, and generating the target DNA; and a step for bringing the reaction solution into a detection condition, and detecting the target DNA.

## Description

### [Technical Field]

The present invention relates to a method and kit for detecting target RNA.

The present application claims the benefit of priority from Japanese Patent Application No. 2023-013023 filed in Japan on January 31, 2023, the contents of which are incorporated herein by reference.

### [Background Art]

In recent years, there is an increasing need to more accurately detect target molecules for the purpose of discovering diseases earlier, and the like. As a method of accurately detecting target molecules, for example, PTL 1, PTL 2, and NPL 1 describe techniques that carry out an enzyme reaction in a large number of micro-compartments. These techniques are referred to as digital measurements.

In a digital measurement, a sample solution is divided into a very large number of microsolutions. Then, a signal from each micro-solution is binarized, and the number of target molecules is measured by only a determination of whether target molecules are present. Conventionally, the quantification of nucleic acids has been carried out by real-time PCR and the like. A digital measurement can significantly improve the detection sensitivity and quantitativeness compared to the conventional real-time PCR method and the like.

In digital PCR, a mixture of a PCR reagent and nucleic acids is diluted such that the number of template nucleic acids present in a single microdroplet becomes 0 or 1. In digital PCR, in order to increase the sensitivity of nucleic acid amplification, and to perform nucleic acid amplification simultaneously for a large number of microdroplets, it is preferable to use a smaller microdroplet volume. PTL 1 discloses a method of measuring a target molecule in a well array having a plurality of wells, each with a depth of 3 µm and a diameter of 5 µm, that are formed in a flow path, and introduces the sample into each well by flowing the sample through the flow path to introduce the samples into the wells, and then pushing out the excess reagent in the flow path using an oil.

Because intracellular RNA is rapidly decomposed when a cell dies, methods have been developed that detect the living cells of a microorganism or the like, and determine whether the cells are alive or dead, by detecting RNA. In particular, the RT-PCR method, in which PCR is carried out after conversion of RNA into DNA by a reverse transcription (RT) reaction is well known (for example, see NPL 2).

In disease diagnosis using target RNA detection, the reverse transcription quantitative PCR method has been used as a gold standard platform. However, in RNA detection by RT-PCR, the DNA produced during the final amplification reaction can sometimes contaminate the following reaction step, inducing a non-specific sequential reaction, which is referred to as carryover. The cross-contamination caused by carryover tends to result in a false positive result, making detection of target RNA difficult. As a result, a step of purifying the RNA, such as DNA decomposition processing, becomes necessary.

NPL 3 describes a platform that combines CRISPR-based nucleic acid detection with a microchamber technique as an amplification-free RNA detection platform. RNA detection requires a nucleic acid cleavage enzyme and a nucleic acid reagent having a highly optimized design. However, enzymes have low single-base discrimination performance, and it is not possible to detect mutant forms in a large quantity of a wild-type form.

### [Citation List]

### [Patent Literature]

PTL 1: JP 6183471 B
PTL 2: JP 2014-503831 T

### [Non-Patent Literature]

NPL 1: Kim S. H., et al., Large-scale femtoliter droplet array for digital counting of single biomolecules, Lab Chip, 2012, 12, 4986-4991, 2012.
NPL 2: Selvaratnam S., et al., Application of reverse transcriptase PCR for monitoring expression of the catabolic dmpN gene in a phenol-degrading sequencing batch reactor, Appl Environ Microbiol. 61 (11), 3981-3985, 1995.
NPL3: Shinoda H., et al., Automated amplification-free digital RNA detection platform for rapid and sensitive SARS-CoV-2 diagnosis, Commun Biol. 5 (1), 473, 2022.

### [Summary of the Invention]

### [Technical Problem]

The detection of target RNA requires a detection method having high sensitivity and high discrimination performance. To perform analysis at a practical level, it is necessary to have excellent features in terms of each of low cost, simplicity of method, short signal detection time, accuracy of detection results, and the like. The present invention has an object of providing a method and kit for detecting target RNA.

### [Solution to Problem]

The present invention includes the following aspects.
[1] A method for detecting target RNA comprising: a step for introducing a reaction solution into a container, the reaction solution containing the target RNA, a reverse transcription reagent that reverse-transcribes the target RNA to generate target DNA, and a detection reagent that detects the target DNA; a step for bringing the reaction solution into a reverse transcription condition, and generating the target DNA; and a step for bringing the reaction solution into a detection condition, and detecting the target DNA.
[2] The method according to [1], wherein the step for detecting the target DNA is carried out by an invasive cleavage assay (ICA).
[3] The method according to [2], wherein the detection reagent that detects the target DNA contains a first single-stranded oligonucleotide (allele probe), a second single-stranded oligonucleotide (ICA oligonucleotide), a third single-stranded oligonucleotide (FRET cassette), and a flap endonuclease, the allele probe and the ICA oligonucleotide each hybridize with the target DNA to form a first triplex structure at a 5' end portion of the allele probe, which is then cleaved by the flap endonuclease that has recognized the first triplex structure to generate a fourth single-stranded oligonucleotide, the FRET cassette is labeled with a fluorescent substance and a quenching substance, forms a hairpin structure on a 5' side by self-hybridization, and has a base sequence on a 3' side that is complementary to that of the fourth single-stranded oligonucleotide, and when the fourth single-stranded oligonucleotide hybridizes on the 3' side, a second triplex structure is formed at the 5' end portion, which is then cleaved by the flap endonuclease that has recognized the second triplex structure, which releases the fluorescent substance and the quenching substance, and emits fluorescence in response to irradiation with excitation light, and the allele probe has a modification at a 3' end portion that prevents nucleotide extension by a reverse transcription reaction.
[4] The method according to [3], wherein the modification that prevents nucleotide extension by the reverse transcription reaction is bonding of an amino group to the 3' end.
[5] The method according to any one of [1] to [4], further comprising a step for sealing the reaction solution in the container with a sealing liquid after the step for introducing the reaction solution, and before the step for generating the target DNA, wherein the sealing liquid is a fluorinated oil or a silicone-based oil, a boiling point of the sealing liquid is 150°C or higher, and a viscosity of the sealing liquid is 2 mm²/s or more.
[6] The method according to any one of [1] to [5], wherein the reverse transcription reagent contains a reverse transcription primer and reverse transcriptase, and the reverse transcription primer binds to the target RNA in a sequence-specific manner.
[7] The method according to [6], wherein a position at which the reverse transcription primer binds to the target RNA in a sequence-specific manner, and a position at which the ICA oligonucleotide hybridizes with the target DNA, are separated by 20 bases or more.
[8] The method according to [6] or [7], wherein the reverse transcriptase contains an enzyme derived from one of a wild-type form, mutant-type form, or improved-type form of Moloney murine leukemia virus, or derived from one of a mutant-type or improved-type of avian myeloblastosis virus.
[9] The method according to any one of [1] to [8], wherein in the reverse transcription reaction condition, a reverse transcription temperature is 20°C or higher and 50°C or lower, and a reaction time is 5 minutes or more and 60 minutes or less.
[10] The method according to any one of [1] to [9], wherein the reaction solution includes magnesium chloride, and a magnesium chloride concentration in the reaction solution is 3 mM or more and 100 mM or less.
[11] The method according to any of [1] to [10], wherein the container is a well, and a volume of the well is 10 fL to 100 pL.
[12] The method according to [11], wherein in the step for introducing the reaction solution, one or fewer molecules of the target RNA is introduced per well.
[13] A kit for detecting target RNA, comprising: a fluidic device provided with a substrate, and a well array arranged on the substrate; a reverse transcription reagent that reverse-transcribes target RNA to generate target DNA; and a detection reagent that detects the target DNA; wherein the detection reagent that detects the target DNA contains a first single-stranded oligonucleotide (allele probe), a second single-stranded oligonucleotide (ICA oligonucleotide), a third single-stranded oligonucleotide (FRET cassette), and a flap endonuclease, the allele probe and the ICA oligonucleotide each hybridize with the target DNA to form a first triplex structure at a 5' end portion of the allele probe, which is then cleaved by the flap endonuclease that has recognized the first triplex structure to generate a fourth single-stranded oligonucleotide, the FRET cassette is labeled with a fluorescent substance and a quenching substance, forms a hairpin structure on a 5' side by self-hybridization, and has a base sequence on a 3' side that is complementary to that of the fourth single-stranded oligonucleotide, and when the fourth single-stranded oligonucleotide hybridizes on the 3' side, a second triplex structure is formed at the 5' end portion, which is then cleaved by the flap endonuclease that has recognized the second triplex structure, which releases the fluorescent substance and the quenching substance, and emits fluorescence in response to irradiation with excitation light, and the allele probe has a modification at a 3' end portion that prevents nucleotide extension by a reverse transcription reaction.
[14] The kit according to [13], wherein the modification that prevents nucleotide extension by the reverse transcription reaction is bonding of an amino group to the 3' end.
[15] The kit according to [13] or [14], further comprising a sealing liquid, wherein the sealing liquid is a fluorinated oil or a silicone-based oil, a boiling point of the sealing liquid is 150°C or higher, and a viscosity of the sealing liquid is 2 mm²/s or more.
[16] The kit according to any one of [13] to [15], wherein the reverse transcription reagent contains a reverse transcription primer and a reverse transcriptase, and the reverse transcription primer binds to the target RNA in a sequence-specific manner.
[17] The kit according to [16], wherein the kit is designed such that a position at which the reverse transcription primer binds to the target RNA in a sequence-specific manner, and a position at which an ICA oligonucleotide hybridizes with the target DNA, are separated by 20 bases or more.
[18] The kit according to [16] or [17], wherein the reverse transcriptase contains an enzyme derived from one of a wild-type form, mutant-type form, or improved-type form of Moloney murine leukemia virus, or derived from one of a mutant-type or improved-type of avian myeloblastosis virus.
[19] The kit according to any one of [13] to [18], wherein a volume per well of the well array is 10 fL to 100 pL.

### [Advantageous Effects of the Invention]

According to the present invention, a method and kit for detecting target RNA can be provided.

### [Brief Description of the Drawings]

Fig. 1 is a schematic diagram illustrating an invasive cleavage assay (ICA) method.
Fig. 2 is a schematic cross-sectional view showing an example of a fluidic device.
Fig. 3 is a schematic cross-sectional view showing an example of a fluidic device.
Fig. 4 is a photograph showing the results of agarose gel electrophoresis in Example 2.
Fig. 5 is a photograph showing the results of agarose gel electrophoresis in Example 3.
Fig. 6 shows graphs of the results of ICA reactions in Example 4.
Fig. 7 shows graphs of the results of ICA reactions in Example 4.
Fig. 8 shows fluorescence microscope images of the results of a reverse transcription-ICA reactions in Example 5.

### [Description of the Embodiments]

Hereinafter, embodiments of the present invention will be described in detail while in some cases referring to the accompanying drawings. Note that, in the drawings, the same or corresponding components are denoted by the same or corresponding reference signs to omit repeated explanations. Further, the drawings are not necessarily to scale, and some of the dimensional ratios are exaggerated for convenience of the illustrations.

### [Method for Detecting Target RNA]

In one embodiment, the present invention provides a method for detecting target RNA including: a step for introducing a reaction solution into a container, the reaction solution containing the target RNA, a reverse transcription reagent that reverse-transcribes the target RNA to generate target DNA, and a detection reagent that detects the target DNA; a step for bringing the reaction solution into a reverse transcription condition, and generating the target DNA; and a step for bringing the reaction solution into a detection condition, and detecting the target DNA.

### (Step for Introducing Reaction Solution)

In this step, a reaction solution containing target RNA, a reverse transcription reagent that reverse-transcribes the target RNA to generate target DNA, and a detection reagent that detects the target DNA, are introduced into a container. The container, the target RNA, the reverse transcription reagent, and the detection reagent will be described later.

### (Step for Reverse-Transcribing Target RNA)

In this step, the reaction solution is brought to a reverse transcription condition, and the target RNA is reverse-transcribed to generate target DNA. The target RNA is RNA representing the detection target. Examples of the target RNA include virus RNA and disease-related RNA, and is not particularly limited. The target RNA may be contained in a biological sample. The biological sample is not particularly limited, and examples include whole blood, serum, plasma, urine, saliva, cells, tissues, viruses, and plant tissues.

As a method for reverse-transcribing the target RNA, a known reverse transcriptase reaction can be used. Examples of reverse transcription reactions specifically include reverse transcription reactions using a reverse transcription kit of the PrimeScript (registered trademark) series or the SuperScript (registered trademark) series. It is particularly preferable to use the PrimeScript (registered trademark) series.

As the reverse transcription primer used in the reverse transcription reaction of the target RNA, it is preferable to use a random primer or a primer that binds to the target RNA in a sequence-specific manner. The primer that binds to the target RNA in a sequence-specific manner may be a PCR primer that amplifies the target DNA that is obtained by reverse-transcribing the target RNA (also referred to as cDNA).

In a case where the step for detecting target DNA is performed by the invasive cleavage assay (ICA) method, it is preferable that the position at which a reverse transcription primer binds to the target RNA in a sequence-specific manner, and the position at which an ICA oligonucleotide is hybridized with the target DNA, are separated by 20 bases or more. When the position at which a reverse transcription primer binds to the target RNA in a sequence-specific manner, and the position at which an ICA oligonucleotide is hybridized with the target DNA, are separated by 20 bases or more, it becomes easier for the allele probe and the ICA oligonucleotide to be hybridized, which increases the base discrimination processing performance, and increases the detection efficiency. It is preferable that the position at which the reverse transcription primer binds to the target RNA in a sequence-specific manner, and a position at which the ICA oligonucleotide is hybridized with the target DNA, are separated by 100 bases or less.

The reverse transcriptase is preferably an enzyme derived from one of a wild-type form, mutant-type form, or improved-type form of Moloney murine leukemia virus (also referred to as MMLV), or derived from a mutant-type or improved-type of avian myeloblastosis virus (also referred to as AMV). MMLV has a high base discrimination processing performance, and a low reverse transcription error rate. AMV has high thermal stability.

In this step, the reaction solution is brought to a reverse transcription condition, and target DNA is generated from the target RNA. Examples of bringing the reaction solution to a detection condition include bringing the temperature of the reaction solution, by heating or cooling, to a temperature at which the reverse transcriptase exhibits activity. The temperature at which the reverse transcriptase exhibits activity is, for example, an isothermal condition at 20°C or higher, preferably 40°C or higher, and more preferably approximately 48°C. In the reverse transcription condition, the reverse transcription temperature is preferably 20°C or higher and 50°C or lower. The time of the reverse transcription reaction is, for example, 5 minutes or more and 60 minutes or less, and is preferably at least 30 minutes, and particularly preferably approximately 60 minutes.

After the reverse transcription reaction, the target RNA and the target DNA (cDNA) are hybridized in some cases. Therefore, it is preferable to separate the target RNA and the target DNA. For example, after the reverse transcription reaction, it is possible to separate the target RNA and the target DNA by maintaining the temperature of the reaction solution at 90°C to 99°C for 1 to 10 minutes to induce thermal denaturation.

### (Step for Detecting Target DNA)

In this step, the target DNA that has been reverse-transcribed is detected. In the method according to the present embodiment, the reverse transcription of the target RNA, and the detection of the reverse-transcribed target DNA are performed in the same container. As the method of detecting the target DNA, any known detection method can be used depending on the characteristics of the target DNA to be detected. Examples of the method include a method of firstly performing, as necessary, a reaction that amplifies the signal derived from the target DNA to a detectable level (also referred to as a signal amplification reaction), and then detecting the amplified signal by using an appropriate means. Here, examples of the signal include fluorescence, chemiluminescence, color development, potential changes, and pH changes.

Examples of the signal amplification reaction include the invasive cleavage assay (ICA) method, such as the Invader (registered trademark) method, the LAMP method, the TaqMan (registered trademark), and the fluorescent probe method. Among these methods, it is particularly preferable to use the ICA method. This is related to the principle of the ICA reaction, in which the signal amplification proceeds by two reaction cycles, namely (1) complementary binding between nucleic acids, and (2) recognition and cleavage of a triplex structure by an enzyme. In such signal amplification reactions, the inhibiting effect imparted by contaminants other than the target molecule on the reaction cycle is small.

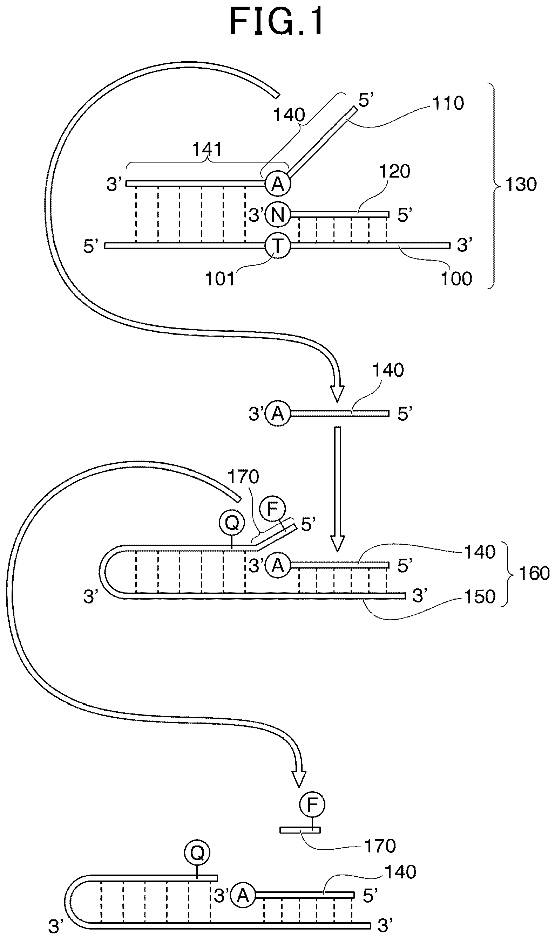

Here, an ICA is described with reference to Fig. 1. Fig. 1 is a schematic diagram illustrating an example of an ICA. In the example of Fig. 1, the presence of target DNA 100 is detected by detecting the presence of a T (thymine) base 101 in the target DNA 100. First, a first single-stranded oligonucleotide (sometimes referred to as an allele probe 110) and a second single-stranded oligonucleotide (sometimes referred to as an ICA oligonucleotide 120) that are complementary to the target DNA 100 are hybridized. As a result, the ICA oligonucleotide 120 is hybridized at a site of the target DNA 100 that is adjacent to the position at which the allele probe 110 is hybridized. Then, at least one base at the 3' end of the ICA oligonucleotide 120 invades the 5' end position of a region 141 in which the allele probe 110 and the target DNA 100 are hybridized, forming a first triplex structure 130.

Then, when the first triplex structure 130 is reacted with a flap endonuclease, a flap site 140 of the first triplex structure 130 is cleaved, producing a fourth single-stranded oligonucleotide (sometimes referred to as a nucleic acid fragment 140). Next, the nucleic acid fragment 140 is hybridized with the third single-stranded oligonucleotide (sometimes also referred to as a FRET cassette 150), forming a second triplex structure 160.

In the example of FIG. 1, a fluorescent substance F is bound to the 5' end of the FRET cassette 150, and a quenching substance Q is bound to the 5' end of the FRET cassette 150 at a position several bases toward the 3' side. The fluorescent substance F is located in spatial proximity to the quenching substance Q. Therefore, the fluorescence emitted by the fluorescent substance F is quenched by the quenching substance Q through fluorescence resonance energy transfer (FRET).

Then, when the second triplex structure 160 is reacted with the flap endonuclease, a flap site 170 of the second triplex structure 160 is cleaved, producing a nucleic acid fragment 170. As a result, the fluorescent substance F becomes free from the quenching substance Q, and emits fluorescence when irradiated with excitation light. As a result of detecting the fluorescence, it is possible to detect the presence of the target DNA (the presence of a thymine (T) base 101 in the target DNA 100).

In a case where the step for detecting target DNA is performed by an ICA, it is preferable that the detection reagent that detects the target DNA contains the first single-stranded oligonucleotide (sometimes referred to as the allele probe), the second single-stranded oligonucleotide (sometimes referred to as the ICA oligonucleotide), the third single-stranded oligonucleotide (sometimes referred to as the FRET cassette), and the flap endonuclease.

As mentioned above, when the allele probe and the ICA oligonucleotide are each hybridized with the target DNA, the first triplex structure is formed on the 5' end portion of the allele probe, which is then cleaved by the flap endonuclease that has recognized the first triplex structure to produce the fourth single-stranded oligonucleotide. Furthermore, the FRET cassette is labeled with a fluorescent substance and a quenching substance, forms a hairpin structure on the 5' side by self-hybridization, and has a base sequence complementary to that of the fourth single-stranded oligonucleotide on the 3' side, and when the fourth single-stranded oligonucleotide is hybridized on the 3' side, the second triplex structure is formed on the 5' end portion. Then, as a result of cleavage by the flap endonuclease that has recognized the second triplex structure, the fluorescent substance and the quenching substance become free from each other, and fluorescence is emitted as a result of irradiation of excitation light.

Here, it is preferable that the allele probe has a modification at the 3' end that prevents nucleotides from being extended by a reverse transcription reaction. As described later in the examples, in a case where the allele probe does not have such a modification, undesired nucleotide extension may occur in the reverse transcription reaction of the target RNA, which can sometimes inhibit detection of the target. In addition, as described later in the examples, in a case where the 3' end of the ICA oligonucleotide has a modification in which nucleotide extension by a reverse transcription reaction is not possible, the ICA reaction itself can sometimes be inhibited.

Examples of modifications that prevent nucleotide extension by a reverse transcription reaction include bonding of an amino group to the 3' end, substitution of an OH group at the 3' end with a hydrogen atom, and substitution of an OH group at the 3' with an azide group (N₃ group).

As the flap endonuclease, flap endonuclease 1 (NCBI accession number: WP_011012561.1), Holliday junction 5' flap endonuclease (GEN1) (NCBI accession number: NP_001123481.3), excision repair protein (NCBI accession number: AAC37533.1), and the like, can be used.

In the present step, the reaction solution is brought to a detection condition, and then the target DNA is detected. Examples of bringing the reaction solution to a detection condition include bringing the temperature of the reaction solution, by heating or cooling, to a temperature at which the ICA reaction proceeds. The temperature at which the ICA reaction proceeds is, for example, an isothermal condition at 60°C or higher, and preferably approximately 66°C. The time of the ICA reaction, for example, is 10 minutes or more, and is preferably approximately 30 minutes.

The reaction solution preferably further includes a salt concentration adjuster. Examples of salt concentration adjusters include sodium chloride and magnesium chloride. In a case where magnesium chloride is used as the salt concentration adjuster, the magnesium chloride concentration contained in the reaction solution is preferably in a range of 3 mM or more and 100 mM or less, and more preferably in a range of 5 mM or more and 50 mM or less. In a typical reverse transcription reaction, the concentration of magnesium chloride is 3 mM. Furthermore, in a typical ICA reaction, the concentration of magnesium chloride is 25 mM.

### (Container)

The method of the present embodiment can be carried out using a digital measurement. In a case where the method of the present embodiment is carried out by the digital measurement, the container is preferably a well, and it is preferable that the well is configured as a well array in which a plurality of wells are arranged. Furthermore, the well array is preferably arranged inside the flow path of the fluidic device. The digital measurement can be performed as a result of introducing one or fewer molecules of the target RNA per well.

### (Fluidic Device)

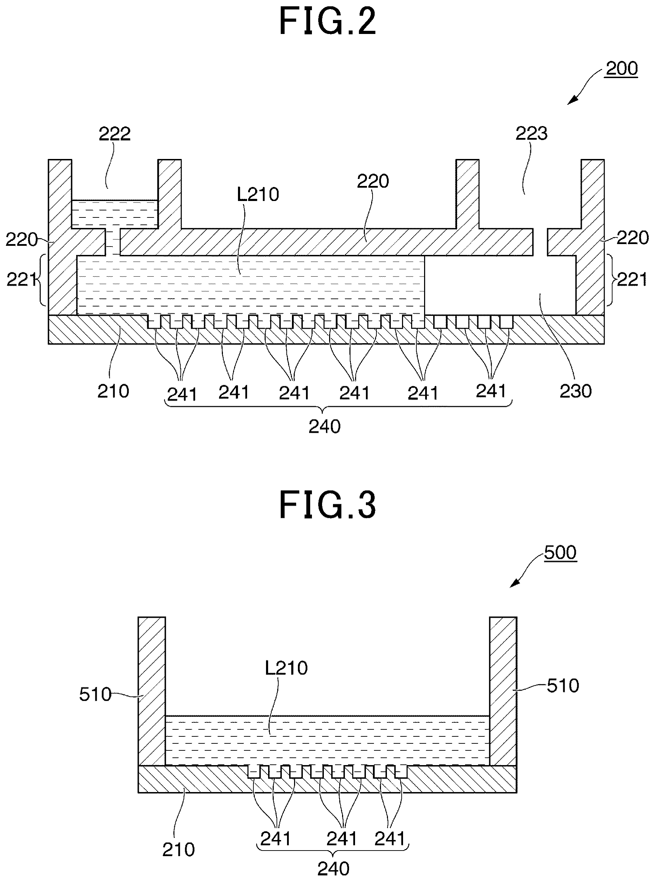

Fig. 2 is a schematic cross-sectional view illustrating an example of a fluidic device with which the method of the present embodiment can be preferably implemented. As shown in Fig. 2, a fluidic device 200 includes a substrate 210, and a lid member 220 disposed facing the substrate 210. The lid member 220 includes a projection portion 221, and the front end of the projection portion 221 makes contact with the substrate 210. In the fluidic device 200, a well array 240 is integrally formed with the substrate 210 on one side of the substrate 210, and faces the lid member 220. The well array 240 includes a plurality of wells 241. The lid member 220 may be welded or adhered to the substrate 210.

The wells 241 are open at the surface of the substrate 210. Although the shape, size, and arrangement of the wells 241 are not specifically limited, the wells 241 are preferably microwells having a small volume. For example, the volume of a single well 241 may be approximately 10 fL to 100 pL. In the fluidic device 200, a plurality of wells 241 having the same shape and size constitute the well array 240. The same shape and size refer to the same shape and the same volume to an extent that is required to perform a digital measurement, and variations of the order of manufacturing errors are acceptable.

The diameter of the wells 241 may, for example, be approximately 1 to 10 µm, and the depth of the wells 241 may, for example, be approximately 1 to 10 µm. The arrangement of the wells 241 is not particularly limited, and may be, for example, arranged in a triangular lattice shape, arranged in a square lattice shape, or may be randomly arranged.

In the fluidic device 200, a space is formed between the well array 240 and the lid member 220 due to the presence of the projection portion 221. The space constitutes a flow path 230. The flow path 230 functions as a passage for a reaction solution L210 containing the target RNA, the reverse transcription reagent, and the detection reagent as mentioned above, and the sealing liquid described later, to be delivered. Although the shape, structure, volume, and the like, of the flow path 230 are not particularly limited, the height of the flow path 230 (the distance between the surface of the substrate 210 and the surface of the lid member 220 facing the substrate 210) may be, for example, 500 µm or less, or for example, 300 µm or less, or for example, 200 µm or less, or for example, 100 µm or less.

The projection portion 221 may be integrally formed with the lid member 220. For example, the lid member 220 can be formed by molding a thermoplastic resin fluid into a plate shape having the projection portion 221 using a mold. The lid member 220 may be provided with an inlet port 222 and a discharge port 223 for a reagent.

In a case where the lid member 220 includes the projection portion 221, the lid member 220 and the substrate 210 are stacked such that the projection portion 221 makes contact with the surface of the substrate 210 at which the wells 241 are open. As a result, the space between the lid member 220 and the substrate 210 serves as the flow path 230. The lid member 220 and the substrate 210 may be welded together by laser welding or the like.

### (Fluidic Device Modification 1)

The fluidic device used in the method of the present embodiment is not limited to the fluidic device 200 described above. Fig. 3 is a schematic cross-sectional view showing an example of a fluidic device. As shown in Fig. 3, the fluidic device 500 includes a substrate 210 and a wall member 510. In the fluidic device 500, the well array 240 is integrated with the substrate 210 on one surface of the substrate 210. The well array 240 includes a plurality of wells 241. The fluidic device 500 mainly differs from the fluidic device 200 described above in that the lid member 220 is not provided.

### (Fluidic Device Modification 2)

In the fluidic device 200 described above, the lid member 220 and the projection portion 221 are integrally formed. However, the lid member 220 and the projection portion 221 may be molded as separate bodies.

Furthermore, in the fluidic device 200 and the fluidic device 500, the well array 240 is integrated with the substrate 210 on one surface of the substrate 210. However, the well array does not have to be integrally formed with the substrate 210. For example, a well array 240 molded as a separate body to the fluidic device may be arranged on the substrate 210 of the fluidic device. Alternatively, a resin layer may be deposited on the surface of the substrate 210, and a well array may be formed in the resin layer by etching or the like.

### (Material of Fluidic Device)

The substrate 210 may be formed using a resin, for example. Although the type of resin is not particularly limited, it is preferable that the resin has resistance to the reagents and the sealing liquid. Furthermore, in a case where the signal to be detected is fluorescence, it is preferable that the resin has low autofluorescence. Examples of the resin include, but are not limited to, cycloolefin polymers, cycloolefin copolymers, silicone, polypropylene, polycarbonate, polystyrene, polyethylene, polyvinyl acetate, fluororesins and amorphous fluororesins.

The substrate 210 may be provided with a plurality of wells 241 formed on one surface in the plate thickness direction. Examples of the method of forming the wells in the resin include injection molding, thermal imprinting, and optical imprinting.

Alternatively, for example, the well array may be formed by laminating a fluororesin on the substrate 210, and then processing the fluororesin by etching or the like. As the fluororesin, for example, CYTOP (registered trademark, AGC Inc.) or the like can be used.

Furthermore, in a case where the fluidic device includes the lid member 220, the material of the lid member 220 is preferably a resin with low autofluorescence, and may be, for example, a thermoplastic resin such as a cycloolefin polymer or a cycloolefin copolymer.

In addition, the lid member 220 may be formed of a material that does not transmit light at wavelengths near the wavelength detected when observing a signal for fluorescence, or may be formed of a material that is completely opaque to light. For example, the lid member 220 may be made of a thermoplastic resin with to which carbon, metal particles, and the like, have been added.

In a case where the method according to the present embodiment is performed using a fluidic device, the reaction solution containing the target RNA, the reverse transcription reagent, and the detection reagent is delivered into an internal space of the fluidic device (for example, the flow path), and is introduced to the wells of the well array. The reaction solution is preferably an aqueous solution. The liquid may be more easily introduced into the wells by adding a surfactant or the like to the reaction solution.

Then, the sealing liquid is delivered through the internal space of the fluidic device (for example, the flow path), and the fluid that has been introduced into the wells is sealed in the wells by forming a layer of the sealing liquid on an upper layer of the fluid. As a result of this step, a microdroplet is formed inside the well, and an independent reaction space is formed in each well. The step for reverse-transcribing target RNA is preferably performed after sealing the reaction solution inside the wells.

Note that, in a case where a fluidic device such as that shown in Fig. 3 is used, the well array may be sealed with the sealing liquid (for example, an oil) by dropwise addition of the reaction solution onto the well array to fill the well array, followed by gentle dropwise addition of the sealing liquid from above.

### (Sealing Liquid)

The sealing solution is a liquid that can individually seal the liquid introduced to the plurality of wells so as to prevent mixing with each other, enable the formation of liquid droplets (microdroplets), and is preferably an oil-based solution, and is more preferably an oil. Examples of the oil that can be used include fluorinated oils, silicone-based oils, hydrocarbon oils, and mixtures thereof.

The boiling point of the sealing liquid is preferably 150°C or higher. The upper limit value of the boiling point of the sealing liquid is, for example, 3,300°C. The boiling point of the sealing liquid is more preferably 150°C or higher, and 3,300°C or lower. The viscosity of the sealing liquid is preferably 2 mm²/s or higher. The upper limit value of the viscosity of the sealing liquid is, for example 300 mm²/s or less. The viscosity of the sealing liquid is more preferably 2 mm²/s or more and 300 mm²/s or less. Among such sealing liquids, the sealing liquid is preferably a fluorinated oil or a silicone-based oil whose boiling point is 150°C or higher, and whose viscosity is 2 mm²/s or more. The viscosity of the sealing liquid can be measured using a capillary viscometer in accordance with the liquid viscosity measurement method in JIS Z 8803:2011.

More specifically, the fluorinated oil having the product name "FC-40" manufactured by Sigma, the fluorinated oil having the product name "FLUO-OIL 40" manufactured by Emulseo, the fluorinated oil having the product name "Fomblim 06/6" manufactured by Solvay, the fluorinated oil having the product name "Galden HT200" manufactured by Solvay, the silicone oil-based oils having the product name "KF-96" manufactured by Shin-Etsu Chemical Co., Ltd., and the like, can be used. FC-40 (CAS Number: 86508-42-1) is a fluorinated aliphatic compound with a specific gravity of 1.85 g/mL at 25°C.

As the signal observation method after the ICA reaction, known methods can be appropriately selected depending on the type of signal to be observed. For example, in a case where bright-field observation is performed, white light is irradiated in a vertical direction with respect to the substrate that is provided with the well array. In a case where fluorescence signal observation is performed, excitation light corresponding to the fluorescent substance is irradiated into the wells from the bottom side of the wells, and the fluorescence emitted from the fluorescent substance is observed. An image of the entire or part of the observed well array is captured and stored, and then image processing is performed by a computer system.

### [Kit for Detecting Target RNA]

In one embodiment, the present invention provides a kit for detecting target RNA, comprising: a fluidic device provided with a substrate, and a well array arranged on the substrate; a reverse transcription reagent that reverse-transcribes target RNA to generate target DNA; and a detection reagent that detects the target DNA; wherein the detection reagent that detects the target DNA contains a first single-stranded oligonucleotide (allele probe), a second single-stranded oligonucleotide (ICA oligonucleotide), a third single-stranded oligonucleotide (FRET cassette), and a flap endonuclease, the allele probe and the ICA oligonucleotide each hybridize with the target DNA to form a first triplex structure at a 5' end portion of the allele probe, which is then cleaved by the flap endonuclease that has recognized the first triplex structure to generate a fourth single-stranded oligonucleotide, the FRET cassette is labeled with a fluorescent substance and a quenching substance, forms a hairpin structure on a 5' side by self-hybridization, and has a base sequence on a 3' side that is complementary to that of the fourth single-stranded oligonucleotide, and when the fourth single-stranded oligonucleotide hybridizes on the 3' side, a second triplex structure is formed at the 5' end portion, which is then cleaved by the flap endonuclease that has recognized the second triplex structure, which releases the fluorescent substance and the quenching substance, and emits fluorescence in response to irradiation with excitation light, and the allele probe has a modification at a 3' end portion that prevents nucleotide extension by a reverse transcription reaction.

The detection method of target RNA described above can be preferably implemented by the kit according to the present embodiment. The kit of the present embodiment may further include a sealing liquid. In the kit according to the present embodiment, the fluidic device, the reverse transcription reagent, the detection reagent, the sealing liquid, the ICA reaction, the allele probe, the ICA oligonucleotide, the FRET cassette, the flap endonuclease, the modification that prevents nucleotide extension by the reverse transcription reaction, and the like, are the same as those described above.

In the kit according to the present embodiment, the modification that prevents nucleotide extension by the reverse transcription reaction may be bonding of an amino group to the 3' end.

In the kit according to the present embodiment, it is preferable that kit further includes a sealing liquid, wherein the sealing liquid is a fluorinated oil or a silicone-based oil, a boiling point of the sealing liquid is 150°C or more, and a viscosity of the sealing liquid is 2 mm²/s or more.

In the kit according to the present embodiment, it is preferable that the reverse transcription reagent contains a reverse transcription primer and a reverse transcriptase, and the reverse transcription primer binds to the target RNA in a sequence-specific manner.

In the kit according to the present embodiment, it is preferable that the kit is designed such that a position at which the reverse transcription primer binds to the target RNA in a sequence-specific manner, and a position at which an ICA oligonucleotide hybridizes with the target DNA are separated by 20 bases or more.

In the kit according to the present embodiment, it is preferable that the reverse transcriptase contains an enzyme derived from one of a wild-type form, mutant-type form, or improved-type form of Moloney murine leukemia virus, or derived from one of a mutant-type or improved-type of avian myeloblastosis virus.

In the kit according to the present embodiment, the volume per well of the well array is preferably 10 fL to 100 pL.

### Examples

The present invention will be described in more detail by way of the following examples, but the present invention is not limited to the following examples.

### [Example 1]

### (Investigation of effect of reverse transcription primer on reverse transcription efficiency)

As the reverse transcription primer, a random 6-mer primer and a reverse transcription primer specific to the target RNA were used, and the reverse transcription efficiency was investigated. Then, 0 fM (negative control), 1.5 fM, or 30 fM of the target RNA (5'-GCCAGGAUGUUUCCUAACGCGCCCUACCUGCCCAGCUGCCUCGAGAGCCAGCCCG CUAUUCGCAAUCAGGGUUACAGCACGGUCACCUUCGACGGGACGCCCAGCUACGG UCACACGCCCUCGCACCAUGCGGCGCAGUU-3', sequence number 1), the reverse transcription primer, and the reverse transcription reaction reagent were mixed, and a reverse transcription reaction was performed using a thermal cycler. The reverse transcription reaction reagent contained reverse transcriptase derived from Moloney murine leukemia virus.

As the reverse transcription primers, a 2.5 µM random 6-mer primer (Takara Bio Inc.), a 0.1 µM reverse transcription primer specific to the target RNA (Fasmac, 5'-AACTGCGCCGCATGGTGC-3', sequence number 2), or 0.1 µM TaqMan PCR antisense primer (Fasmac, 5'-AACTGCGCCGCATGG-3', sequence number 3) were used.

The reverse transcription reaction was carried out at 42°C for 60 minutes, 95°C for 5 minutes, and 4°C for 5 minutes. After the reverse transcription reaction, 1 µL of RNase H was added to the reaction solution, and the reaction was carried out at 37°C for 20 minutes using a thermal cycler.

Then, the reaction solution after the reverse transcription reaction or target DNA for creating a calibration curve was mixed with the TaqMan PCR reagent, and a quantitative real-time PCR reaction was performed using a LightCycler-LC480 (Roche). For the quantitative real-time PCR reaction, a TaqMan PCR primer (sense primer: 5'-CGCAATCAGGGTTACAGCAC-3', sequence number 4; antisense primer: 5'-AACTGCGCCGCATGG-3', sequence number 3) and a TaqMan probe (5'-(VIC)CGTCCCGTCGAAGGTGAC(MGB)-3', sequence number 5) were used. The quantitative real-time PCR reaction was carried out for 70 cycles, with a single cycle being 98°C for 10 seconds and 68°C for 60 seconds.

As a result, when the reverse transcription reaction was performed using the random 6-mer primer, 45.4 fM of cDNA was synthesized from 1.5 pM of target RNA, and the reverse transcription efficiency was calculated to be 3.0%. In addition, 0.91 pM of cDNA was synthesized from 30 pM of target RNA, and the reverse transcription efficiency was calculated to be 3.0%.

In contrast, when the reverse transcription reaction was performed using the sequence-specific primer, 0.4 pM of cDNA was synthesized from 1.5 pM of target RNA, and the reverse transcription efficiency was calculated to be 26.8%. Also, 8.97 pM of cDNA was synthesized from 30 pM of target RNA, and the reverse transcription efficiency was calculated to be 29.9%.

Moreover, when the reverse transcription reaction was performed using the TaqMan PCR primer, 0.78 pM of cDNA was synthesized from 1.5 pM of target RNA, and the reverse transcription efficiency was calculated to be 52.1%. In addition, 17.2 pM of cDNA was synthesized from 30 pM of target RNA, and the reverse transcription efficiency was calculated to be 57.3%.

From the above results, it was shown that, in the RNA reverse transcription reaction, the reverse transcription efficiency was higher when the sequence-specific primer and the TaqMan PCR primer were used than when the random 6-mer primer was used.

### [Example 2]

### (Investigation of Effect of ICA Reagent on RNA Reverse Transcription Reaction)

The reverse transcription reaction was performed in the presence of, and in the absence of, the ICA reagent, and the effect of the ICA reagent on the RNA reverse transcription reaction was investigated. Then, 0 fM (negative control), 1.5 fM, or 30 fM of the target RNA (5'-GCCAGGAUGUUUCCUAACGCGCCCUACCUGCCCAGCUGCCUCGAGAGCCAGCCCG CUAUUCGCAAUCAGGGUUACAGCACGGUCACCUUCGACGGGACGCCCAGCUACGG UCACACGCCCUCGCACCAUGCGGCGCAGUU-3', sequence number 1), the reverse transcription primer, the reverse transcription reaction reagent, and the ICA reagent were mixed, and a reverse transcription reaction was performed using a thermal cycler. The reverse transcription reaction reagent contained reverse transcriptase derived from Moloney murine leukemia virus.

As the reverse transcription primer, a 2.5 µM random 6-mer primer (Takara Bio Inc.) was used. As the ICA reagents, a sample using 2 µM of an allele probe (Fasmac, 5'-CGCGCCGAGGGTTACAGCACGGTCA-3', sequence number 6), 1 µM of an ICA oligonucleotide (Fasmac, 5'-CCTCGAGAGCCAGCCCGCTATTCGCAATCAGGA-3', sequence number 7), and 4 µM of a FRET cassette (Alexa488-BHQ, Japan Bio Services, 5'-(Alexa488)TTCTT(BHQ1)AGCCGGTTTTCCGGCTGAGACCTCGGCGCG-3', sequence number 8), and a sample using 0.2 mg/mL of a flap endonuclease were each prepared.

The reverse transcription reaction was carried out at 42°C for 60 minutes, 95°C for 5 minutes, and 4°C for 5 minutes. Then, the reaction solution after the reverse transcription reaction and the PCR reagent were mixed, and a PCR reaction was carried out using a thermal cycler. In the PCR reaction, a sense primer (5'-CGCAATCAGGGTTACAGCAC-3', sequence number: 9) and an antisense primer (5'-GTGCGAGGGCGTGTGACC-3', sequence number: 10) were used. The PCR reaction was carried out for 30 cycles, with a single cycle being 98°C for 10 seconds, 65°C for 15 seconds, and 68°C for 60 seconds.

Then, each of the reaction solutions after the PCR reaction were mixed with a loading buffer, loaded onto a 4% PrimeGel Agarose gel (Takara Bio Inc.), and then subjected to electrophoresis at 100 V for approximately 40 minutes. Next, the gels after electrophoresis were immersed in a 10,000-times diluted SYBR Gold solution, and then stained for 30 minutes while being stirred with a rotator. Then, the stained gel was imaged using a gel imaging analysis device.

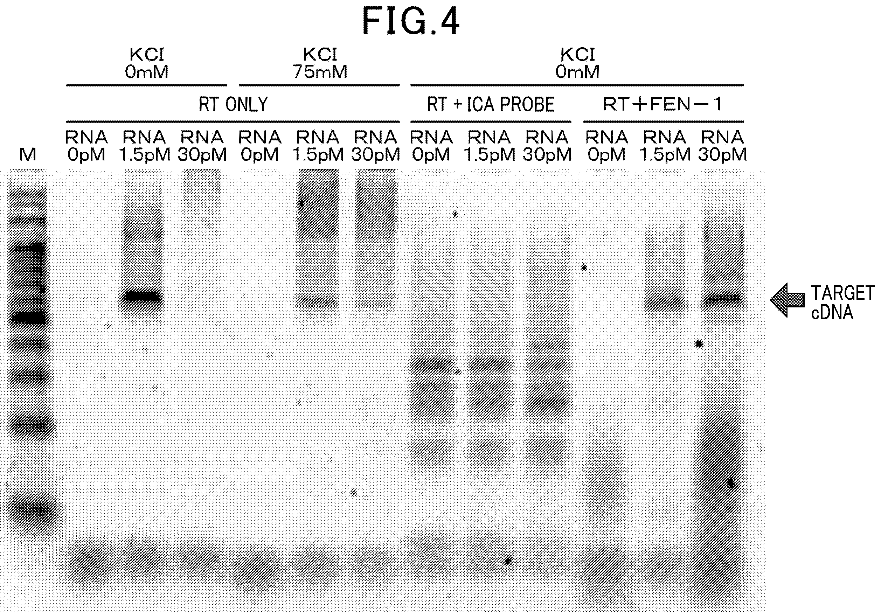

Fig. 4 is a photograph showing the results of agarose gel electrophoresis. In Fig. 4, "M" indicates the marker, "RT only" indicates the result of carrying out the reverse transcription reaction in the absence of the ICA reagent, "RT + ICA probe" indicates the result of carrying out the reverse transcription reaction in the presence of the allele probe, the ICA oligonucleotide, and the FRET cassette, and "RT + FEN-1" indicates the result of carrying out the reverse transcription reaction in the presence of the flap endonuclease. For "RT only", the reverse transcription reaction was carried out with KCl concentrations of 0 mM and 75 mM. For "RT + ICA probe" and "RT + FEN-1", the reverse transcription reaction was carried out with a KCl concentration of 0 mM. Furthermore, the arrow indicates the position of the target cDNA band.

As a result, in the reverse transcription reaction of the target RNA, in those cases where the reverse transcription reaction was carried out in the presence of the allele probe, the ICA oligonucleotide, and the FRET cassette, a band was observed in a different position to those cases where the reverse transcription reaction was carried out in the absence of the ICA reagent. This result indicates that the allele probe, the ICA oligonucleotide, and the FRET cassette are affecting the reverse transcription reaction.

### [Example 3]

### (Investigation of Effect of ICA Reagent on PCR Reaction)

In order to investigate the results of Example 2 in more detail, the effect of the ICA reagent on a PCR reaction using cDNA obtained by reverse-transcribing the target RNA (base sequence is represented by sequence number 1) as a template was investigated. A DNA fragment having the same base sequence as the cDNA above (5'-AACTGCGCCGCATGGTGCGAGGGCGTGTGACCGTAGCTGGGCGTCCCGTCGAAGGT GACCGTGCTGTAACCCTGATTGCGAATAGCGGGCTGGCTCTCGAGGCAGCTGGGCA GGTAGGGCGCGTTAGGAAACATCCTGGC-3', sequence number 11) was mixed with the PCR reagent, and the PCR reaction was carried out using a thermal cycler. For the PCR reaction, a combination of a sense primer (5'-CGCAATCAGGGTTACAGCAC-3', sequence number 9) and an antisense primer (5'-GTGCGAGGGCGTGTGACC-3', sequence number 10), or a TaqMan PCR primer (sense primer: 5'-CGCAATCAGGGTTACAGCAC-3', sequence number 4; antisense primer: 5'-AACTGCGCCGCATGG-3', sequence number 3) were used. The PCR reaction was carried out for 30 cycles, with a single cycle being 98°C for 10 seconds, 65°C for 15 seconds, and 68°C for 60 seconds.

As the ICA reagent, an allele probe modified with an amino group at the 3' end (5'-CGCGCCGAGGGTTACAGCACGGTCA-3', sequence number 6) and an allele probe not modified with an amino group (Fasmac, 5'-CGCGCCGAGGGTTACAGCACGGTCA-3', sequence number 6) were used.

Then, each of the reaction solutions after the PCR reaction were mixed with a loading buffer, loaded onto a 4% PrimeGel Agarose gel (Takara Bio Inc.), and then subjected to electrophoresis at 100 V for approximately 40 minutes. Next, the gels after electrophoresis were immersed in a 10,000-times diluted SYBR Gold solution, and then stained for 30 minutes while being stirred with a rotator. Then, the stained gel was imaged using a gel imaging analysis device.

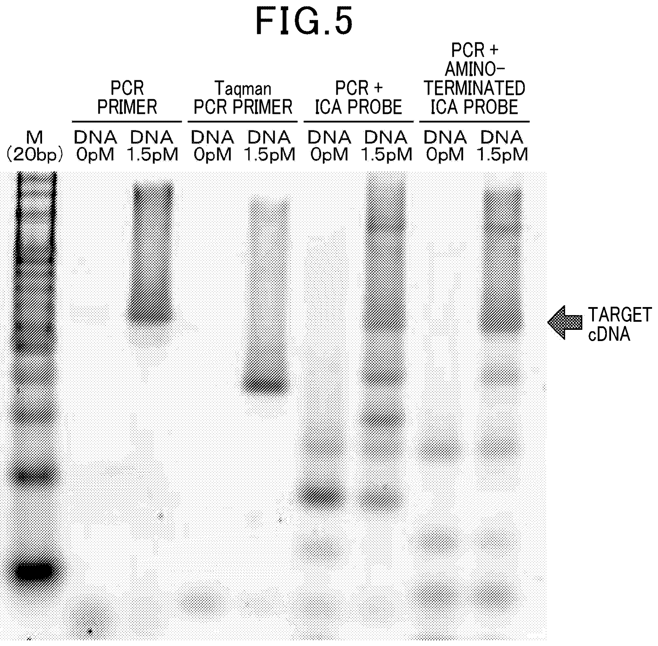

Fig. 5 is a photograph showing the results of agarose gel electrophoresis. In Fig. 5, "M" indicates the marker, "PCR primer" indicates the result of carrying out the PCR reaction in the absence of the ICA reagent and using the primers with base sequences represented by sequence numbers 9 and 10, "TaqMan PCR primer" indicates the result of carrying out the PCR reaction in the absence of the ICA reagent using the primers with base sequences represented by the sequence numbers 3 and 4, "PCR + ICA probe" indicates the result of carrying out the PCR reaction in the presence of an allele probe that has not been modified by an amino group using the primers with base sequences represented by sequence numbers 9 and 10, "PCR + amino-terminated ICA probe" represents the result of carrying out the PCR reaction in the presence of an allele probe in which the 3' end has been modified with an amino group using the primers with base sequences represented by sequence numbers 9 and 10, and the arrow indicates the band position of the target DNA.

As a result, it was confirmed that, in a case where the PCR reaction was carried out in the presence of an allele probe in which the 3' end has been modified with an amino group, compared to those cases where the PCR reaction was carried out in the presence of an allele probe that has not been modified with an amino group, the target DNA band was darker, and several bands other than the target DNA band disappeared. This result indicates that the effect on the PCR reaction was reduced due to modifying the 3' end of the allele probe with an amino group. In the PCR reaction, DNA polymerase extends the DNA strand from the 3' end. For this reason, it is thought that modifying the 3' end with an amino group can inhibit the elongation of DNA strands other than those of the target.

### [Example 4]

### (Investigation of Effect of End-Modified ICA Probe on ICA Reaction)

Here, 0 pM (negative control), 1.5 pM, or 30 pM of the target DNA (5'-AACTGCGCCGCATGGTGCGAGGGCGTGTGACCGTAGCTGGGCGTCCCGTCGAAGGT GACCGTGCTGTAACCCTGATTGCGAATAGCGGGCTGGCTCTCGAGGCAGCTGGGCA GGTAGGGCGCGTTAGGAAACATCCTGGC-3', sequence number 11), a reverse transcription reaction reagent containing reverse transcriptase derived from Moloney murine leukemia virus, and an ICA reagent were mixed, and the PCR reaction was carried out using a thermal cycler.

As the ICA reagent, 2 µM of an allele probe in which the 3' end was modified with an amino group or not modified with an amino group (Fasmac, 5'-CGCGCCGAGGGTTACAGCACGGTCA-3', sequence number 6), 1 µM of an ICA oligonucleotide in which the 3' end was modified with an amino group or not modified with an amino group (Fasmac, 5'-CCTCGAGAGCCAGCCCGCTATTCGCAATCAGGA-3', sequence number 7), and 4 µM of a FRET cassette (Alexa488-BHQ, Japan Bio Services, 5'-(Alexa488)TTCTT(BHQ1)AGCCGGTTTTCCGGCTGAGACCTCGGCGCG-3', sequence number 8), and a flap endonuclease was used. The mixed reagent was dispensed into a 96-well plate, and the ICA reaction was carried out using a LightCycler LC480 (Roche). The ICA reaction was carried out at a constant 65°C.

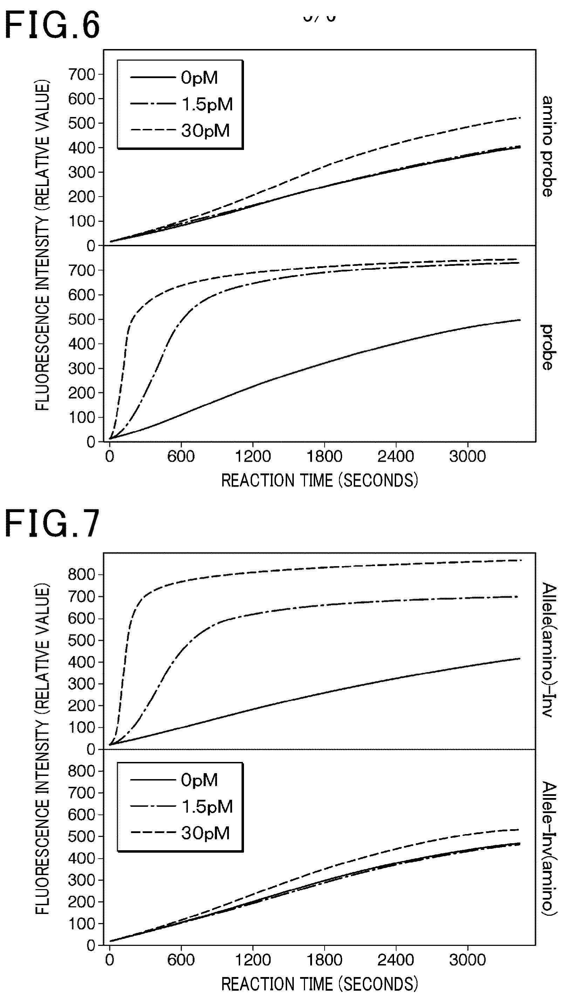

Figs. 6 and 7 are graphs showing the results of the ICA reactions. In Figs. 6 and 7, the vertical axis represents the fluorescence intensity (relative value) detected as a result of the ICA reaction, and the horizontal axis represents the reaction time (seconds).

The upper part of Fig. 6 shows the results using an allele probe in which the 3' end was modified with an amino group and an ICA oligonucleotide in which the 3' end was modified with an amino group, and the lower part of Fig. 6 shows the results using an allele probe that was not modified with an amino group and an ICA oligonucleotide that was not modified with an amino group.

As a result, in a case where an allele probe in which the 3' end was modified with an amino group and an ICA oligonucleotide in which the 3' end was modified with an amino group were used, no difference was observed between the results obtained using DNA concentrations of 1.5 pM and 30 pM and the background result using a DNA concentration of 0 pM, making it clear that the target DNA cannot be detected by the ICA reaction.

In contrast, in a typical case where an allele probe that was not modified with an amino group and an ICA oligonucleotide that was not modified with an amino group were used, a difference was observed between the results obtained using DNA concentrations of 1.5 pM and 30 pM and the background result using a DNA concentration of 0 pM, making it clear that the target DNA can be detected by the ICA reaction.

From these results, it is thought that the allele probe in which the 3' end was modified with an amino group or the ICA oligonucleotide in which the 3' end was modified with an amino group inhibited the ICA reaction.

The upper part of Fig. 7 shows the results using an allele probe in which the 3' end was modified with an amino group and an ICA oligonucleotide that was not modified with an amino group, and the lower part of Fig. 7 shows the results using an allele probe that was not modified with an amino group and an ICA oligonucleotide in which the 3' end was modified with an amino group.

As a result, in a case where an allele probe in which the 3' end was modified with an amino group and an ICA oligonucleotide in which the 3' end was modified with an amino group were used, a difference was observed between the results obtained using DNA concentrations of 1.5 pM and 30 pM and the background result using a DNA concentration of 0 pM, making it clear that the target DNA can be detected by the ICA reaction.

In contrast, in a case where an allele probe that was not modified with an amino group and an ICA oligonucleotide in which the 3' end was modified with an amino group were used, no difference was observed between the results obtained using DNA concentrations of 1.5 pM and 30 pM and the background result using a DNA concentration of 0 pM, making it clear that the target DNA cannot be detected by the ICA reaction.

From the above results, it is considered possible that, by using an allele probe that is not modified with an amino group and an ICA oligonucleotide in which the 3' end is modified with an amino group, the ICA reaction can be performed with a reduced effect of the ICA reagent on the reverse transcription reaction.

### [Example 5]

### (Fluidic Device Using Reverse Transcription-ICA Reaction)

First, a fluidic device was produced. A base material and a lid material (colored with addition of carbon black, and equipped with liquid delivery and liquid discharge ports) made of a cycloolefin polymer formed by injection molding were bonded together to form an internal space (flow path) having a height of 30 µm. A well array was formed on the substrate, and the wells had a diameter of 5 or 10 µm and a volume that allowed signal detection by the ICA reaction within several minutes.

Three fluidic devices were prepared. Ethanol was delivered to each fluidic device using a syringe. Then, a loading buffer was delivered. Next, solutions containing 0 fM (negative control), 20 fM, or 200 fM of the target RNA (5'-GCCAGGAUGUUUCCUAACGCGCCCUACCUGCCCAGCUGCCUCGAGAGCCAGCCCG CUAUUCGCAAUCAGGGUUACAGCACGGUCACCUUCGACGGGACGCCCAGCUACGG UCACACGCCCUCGCACCAUGCGGCGCAGUU-3', sequence number 1) mixed with a reverse transcription-ICA reaction reagent were each delivered. Then, a sealing liquid FC-40 (CAS number: 86508-42-1, Sigma) was delivered to seal each well of each fluidic device. As a result, each well of the well array became an independent reaction space.

In the present example, a reverse transcription-ICA reaction represents a reverse transcription reaction and a subsequent ICA reaction. As the reverse transcription-ICA reagent, 0.1 µM of a reverse transcription primer specific to the target RNA (Fasmac, 5'-AACTGCGCCGCATGGTGC-3', sequence number: 2), reverse transcriptase derived from Moloney murine leukemia virus, 2 µM of an allele probe in which the 3' end was modified with an amino group (Fasmac, 5'-CGCGCCGAGGGTTACAGCACGGTCA-3', sequence number 6), 0.05 µM of an ICA oligonucleotide that was not modified with an amino group (Fasmac, 5'-CCTCGAGAGCCAGCCCGCTATTCGCAATCAGGA-3', sequence number 7), and 4 µM of a FRET cassette (Alexa488-BHQ, Japan Bio Services, 5'-(Alexa488)TTCTT(BHQ1)AGCCGGTTTTCCGGCTGAGACCTCGGCGCG-3', sequence number 8), and a flap endonuclease was used.

Then, the fluidic device in which each well was sealed was placed on a hot plate, and the reverse transcription-ICA reaction was carried out. The reverse transcription reaction was carried out at 48°C for 60 minutes, 95°C for 5 minutes, and 4°C for 5 minutes. The ICA reaction was carried out after the reverse transcription reaction. The ICA reaction was carried out at 66°C for 30 minutes.

Then, each fluidic device was cooled and then observed under a fluorescence microscope (BZ-710, Keyence Corporation). A 4x objective was used to capture bright-field images of each well of the well array, as well as fluorescent images of the fluorescence obtained in the ICA detection reaction.

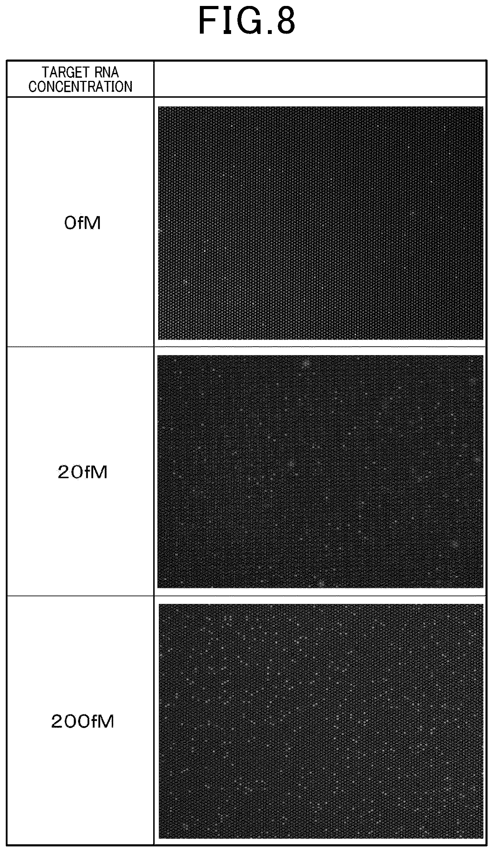

Fig. 8 shows fluorescence microscope images. As a result, it was confirmed that the number of detected wells at target RNA concentrations of 20 fM and 200 fM increased compared to the number of detected wells at a target RNA concentration of 0 fM.

This result indicates that cDNA was transcribed by the reverse transcription reaction of target RNA inside the wells of the fluidic device, and further, that an ICA reaction could be carried out continuously.

### [Industrial Applicability]

According to the present invention, a method and kit for detecting target RNA can be provided.

### [Reference Signs List]

- 100: Target DNA,
- 101: Base,
- 110: Allele probe,
- 120: ICA oligonucleotide,
- 130: First triplex structure,
- 140, 170: Flap site (nucleic acid fragment),
- 141: Region,
- 150: FRET cassette,
- 151: Mismatch site,
- 160: Second triplex structure,
- 200, 500: Fluidic device,
- 210: Substrate,
- L210: Reaction solution,
- 220: Lid member,
- 221: Projection portion,
- 222: Inlet port,
- 223: Discharge port,
- 230: Flow path,
- 240: Well array,
- 241: Well,
- F: Fluorescent substance,
- Q: Quenching substance.

## Claims

1. A method for detecting target RNA comprising:
a step for introducing a reaction solution into a container, the reaction solution containing the target RNA, a reverse transcription reagent that reverse-transcribes the target RNA to generate target DNA, and a detection reagent that detects the target DNA;
a step for bringing the reaction solution into a reverse transcription condition, and generating the target DNA; and
a step for bringing the reaction solution into a detection condition, and detecting the target DNA.

2. The method according to claim 1, wherein
the step for detecting the target DNA is carried out by an invasive cleavage assay.

3. The method according to claim 2, wherein
the detection reagent that detects the target DNA contains an allele probe, being a first single-stranded oligonucleotide, an ICA oligonucleotide, being a second single-stranded oligonucleotide, a FRET cassette, being a third single-stranded oligonucleotide, and a flap endonuclease,
the allele probe and the ICA oligonucleotide each hybridize with the target DNA to form a first triplex structure at a 5' end portion of the allele probe, which is then cleaved by the flap endonuclease that has recognized the first triplex structure to generate a fourth single-stranded oligonucleotide,
the FRET cassette is labeled with a fluorescent substance and a quenching substance, forms a hairpin structure on a 5' side by self-hybridization, and has a base sequence on a 3' side that is complementary to that of the fourth single-stranded oligonucleotide, and when the fourth single-stranded oligonucleotide hybridizes on the 3' side, a second triplex structure is formed at the 5' end portion, which is then cleaved by the flap endonuclease that has recognized the second triplex structure, which releases the fluorescent substance and the quenching substance, and emits fluorescence in response to irradiation with excitation light, and
the allele probe has a modification at a 3' end portion that prevents nucleotide extension by a reverse transcription reaction.

4. The method according to claim 3, wherein
the modification that prevents nucleotide extension by the reverse transcription reaction is bonding of an amino group to the 3' end.

5. The method according to any one of claims 1 to 4, further comprising
a step for sealing the reaction solution in the container with a sealing liquid after the step for introducing the reaction solution, and before the step for generating the target DNA, wherein
the sealing liquid is a fluorinated oil or a silicone-based oil,
a boiling point of the sealing liquid is 150°C or higher, and
a viscosity of the sealing liquid is 2 mm²/s or more.

6. The method according to any one of claims 1 to 4, wherein
the reverse transcription reagent contains a reverse transcription primer and reverse transcriptase, and
the reverse transcription primer binds to the target RNA in a sequence-specific manner.

7. The method according to claim 6, wherein
a position at which the reverse transcription primer binds to the target RNA in a sequence-specific manner, and a position at which the ICA oligonucleotide hybridizes with the target DNA are separated by 20 bases or more.

8. The method according to claim 6, wherein
the reverse transcriptase contains an enzyme derived from one of a wild-type form, mutant-type form, or improved-type form of Moloney murine leukemia virus, or derived from one of a mutant-type or improved-type of avian myeloblastosis virus.

9. The method according to any one of claims 1 to 4, wherein
in the reverse transcription condition, a reverse transcription temperature is 20°C or higher and 50°C or lower, and a reaction time is 5 minutes or more and 60 minutes or less.

10. The method according to any one of claims 1 to 4, wherein
the reaction solution includes magnesium chloride, and a magnesium chloride concentration in the reaction solution is 3 mM or more and 100 mM or less.

11. The method according to any one of claims 1 to 4, wherein
the container is a well, and a volume of the well is 10 fL to 100 pL.

12. The method according to claim 11, wherein
in the step for introducing the reaction solution, one or fewer molecules of the target RNA is introduced per well.

13. A kit for detecting target RNA, comprising:
a fluidic device provided with a substrate, and a well array arranged on the substrate;
a reverse transcription reagent that reverse-transcribes target RNA to generate target DNA; and
a detection reagent that detects the target DNA; wherein
the detection reagent that detects the target DNA contains an allele probe, being a first single-stranded oligonucleotide, an ICA oligonucleotide, being a second single-stranded oligonucleotide, a FRET cassette, being a third single-stranded oligonucleotide, and a flap endonuclease,
the allele probe and the ICA oligonucleotide each hybridize with the target DNA to form a first triplex structure at a 5' end portion of the allele probe, which is then cleaved by the flap endonuclease that has recognized the first triplex structure to generate a fourth single-stranded oligonucleotide,
the FRET cassette is labeled with a fluorescent substance and a quenching substance, forms a hairpin structure on a 5' side by self-hybridization, and has a base sequence on a 3' side that is complementary to that of the fourth single-stranded oligonucleotide, and when the fourth single-stranded oligonucleotide hybridizes on the 3' side, a second triplex structure is formed at the 5' end portion, which is then cleaved by the flap endonuclease that has recognized the second triplex structure, which releases the fluorescent substance and the quenching substance, and emits fluorescence in response to irradiation with excitation light, and
the allele probe has a modification at a 3' end portion that prevents nucleotide extension by a reverse transcription reaction.

14. The kit according to claim 13, wherein
the modification that prevents nucleotide extension by the reverse transcription reaction is bonding of an amino group to the 3' end.

15. The kit according to claim 13, further comprising
a sealing liquid, wherein
the sealing liquid is a fluorinated oil or a silicone-based oil,
a boiling point of the sealing liquid is 150°C or higher, and
a viscosity of the sealing liquid is 2 mm²/s or more.

16. The kit according to claim 13, wherein
the reverse transcription reagent contains a reverse transcription primer and a reverse transcriptase, and
the reverse transcription primer binds to the target RNA in a sequence-specific manner.

17. The kit according to claim 16, wherein
the kit is designed such that a position at which the reverse transcription primer binds to the target RNA in a sequence-specific manner, and a position at which an ICA oligonucleotide hybridizes with the target DNA are separated by 20 bases or more.

18. The kit according to claim 16, wherein
the reverse transcriptase contains an enzyme derived from one of a wild-type form, mutant-type form, or improved-type form of Moloney murine leukemia virus, or derived from one of a mutant-type or improved-type of avian myeloblastosis virus.

19. The kit according to any one of claims 13 to 18, wherein
a volume per well of the well array is 10 fL to 100 pL.
